# EUROPEAN PATENT SPECIFICATION

(11) **EP 1 677 753 B1**
(45) Date of publication and mention of the grant of the patent: **12.08.2009**
(21) Application number: 04765354.8
(22) Date of filing: 16.09.2004
(51) Int. Cl.: A61K 8/92, A61K 8/89, A61Q 5/02, A61Q 5/12

(54) **HAIR CARE COMPOSITION**
HAARPFLEGEZUSAMMENSETZUNG
COMPOSITION POUR LES SOINS CAPILLAIRES

(30) Priority: 27.10.2003 EP 03256770; 03.02.2004 GB 0402270
(43) Date of publication of application: 12.07.2006
(73) Proprietor: Unilever PLC, London EC4Y 0DY (GB); Unilever N.V., 3013 AL Rotterdam (NL)
(72) Inventor: MAHADESHWAR, Anand R., Unilever R&D PortSunlight, Bebington, Wirral, Merseyside CH63 3JW (GB)
(74) Representative: Tansley, Sally Elizabeth
(86) International application number: PCT/EP2004/010462
(87) International publication number: WO 2005/046628

(56) References cited:
- EP-A- 0 502 616
- US-A1- 2003 143 177
- US-A1- 2003 171 479

## Description

### Technical Field

The invention is concerned with improvements to hair conditioning compositions. Such compositions have, as one of their aims, the improvement of the condition of the hair when wet or dry, by which is meant properties such as ease of combing, detangling, smoothness and softness of the hair.
The conditioning effect may be a benefit present in addition to the main purpose of the composition (such as for shampoos, styling products or hair dye systems), or it may be the main purpose of the composition, such as for rinse-off hair conditioners or leave-in conditioners and mousses.
The invention is particularly concerned with conditioning shampoos or shower gels, which are used to wash the hair, removing grease and soil, and which also provide conditioning benefits to the hair.

### Background to the Invention

Consumers from different backgrounds, cultures and ethnic origins have differing preferences for hair conditioning.
Style and fashion considerations can also lead to changes in the desired condition of the hair. One group of consumers desires hair which is straighter and easier to manage after treatment: by this is meant reduced hair volume, less fluffiness and greater mutual alignment of the hairs.

One way to achieve this is to apply adhesive conditioning materials such as high molecular weight polymers such as silicones (polydialkylsiloxanes) or hydrocarbon oils or waxes to the hair. Although the presence of such materials in compositions may lead to the desired attributes of reduced hair volume, less fluffiness and greater mutual alignment, it also may lead to problems of sensory negatives as it can leave the hair feeling coated, greasy and sticky.

Moreover, water-insoluble conditioning materials are generally suspended in compositions, particularly shampoo compositions, as particles or droplets, typically less than 50 micrometres in diameter. This is in order to achieve deposition of conditioning particles or droplets onto the hair, and to ensure that the product remains relatively stable to separation of the ingredients on storage. In order to achieve suspension of the conditioning material, shampoos are sometimes designed to have a shear-thinning viscosity profile such that they are viscous at low shear rates, so that the conditioning material is suspended, but less viscous at higher shear rates, such that the shampoo can be poured from the bottle. The desired viscosity profile for the composition may be achieved by use of viscosity modifiers such as polymers, but can also be achieved by tailoring the micellar phase structure of the shampoo using electrolytes. Some adhesive conditioning materials such as mineral oils and waxes and ester oils can interact with the phase structure of the shampoo during long term storage, leading to loss of structural stability and consequent physical separation of ingredients.

It has now been found that by using a combination of a high viscosity silicone polymer and beeswax in an aqueous hair treatment composition, the conditioning effects of straightening and manageability can be achieved while minimising the problems of negative, coated feel and stickiness. Moreover, it has also been found that the beeswax and silicone polymer combination minimises problems of separation of ingredients on storage in surfactant-structured compositions, particularly for shampoos and rinse-off conditioner compositions.

Documents US 2003/143177 and US 2003/171479 describe hair conditioning compositions containing high viscosity polysiloxanes and waxes.

Document EP-A-502616 discloses the use of beeswax in conditioning shampoos.

### Summary of the Invention

In a first aspect, the invention provides an aqueous hair conditioning composition comprising:
a) 0.1% to 8% by:weight of the total composition of beeswax
b) 0.01% to 10 % by weight of the total composition of a silicone polymer with a viscosity at 0.01Hz of at least 80,000 mm²/s at 25°C.

In a second aspect, the invention is concerned with a process for preparing the hair care composition discussed above comprising the step of emulsifying the beeswax before addition to the composition.

In a third aspect the invention is concerned with the use of the composition discussed above for conditioning hair.

### Detailed Description of the Invention

By water insoluble it is meant that a material has a solubility in water of 0.1% or less by weight of water at 25°C. By non-volatile it is meant that a material has a vapour pressure of less than 1000 Pa at 25°C.

Viscosities, except where otherwise specified, are dynamic viscosities. These may be measured using a cone and plate rheometer at 25°C and at a shear rate of 0.01s⁻¹.

Where particles are referred to in the description, the broad definition of particles is meant, indicating that a material is present in a divided form. If the material is a liquid, the particles will be in the form of droplets.

Particle sizes are suitably measured by laser light scattering using an instrument such as a Malvern^{™} Mastersizer. Particle diameters are expressed as median particle diameters (D₅₀).

### Aqueous Composition

Compositions according to the invention comprise water. Suitably compositions according to the invention comprise 60 or more, preferably 65 or more, more preferably 70 or more percent by weight of water.

### Beeswax

Beeswax is secreted by bees from glands under their abdomen. It is used by the bees to construct the honeycomb. The wax is available as a commercial by-product of the harvesting and refining of honey. The beeswax is used in the invention preferably in a particulate form as particles with a median (D₅₀) diameter of 50 micrometres or less, preferably 20 micrometres or less, more preferably 10 micrometres or less and even more preferably 1 micrometre or less.

The beeswax is present from 0.1% to 8% by weight of the total composition, preferably from 0.2 to 5 wt%, more preferably from 0.4 % to 3%, most preferably from 0.6% to 2%.

The beeswax may be pre-formed into an emulsion or dispersion before addition to the rest of the composition. Preferably the beeswax is pr-formed into an emulsion

A preferred process for incorporating the beeswax into the composition comprises the steps of (i) heating the composition without beeswax to a temperature of 65°C or higher, preferably 75°C or higher, (ii) melting the beeswax, (iii) combining the beeswax and the rest of the composition while stirring and (iv) cooling the composition to room temperature, typically 25°C.

Surprisingly, the beeswax is self-emulsifying without the need for vigorous agitation if such a process is followed.

An alternative preferred process for incorporating beeswax into the composition involves the following steps:
i) Preparing an aqueous solution or dispersion of emulsifier at a temperature of 65°C or more, preferably 70°C or more, more preferably 80 °C or more.
ii) Preparing molten beeswax at a temperature of 65°C or more, preferably 70°C or more, more preferably 80 °C or more.
iii) Mixing and homogenising the liquids of steps (i) and (ii).
iv) Cooling the resulting dispersion to room temperature while stirring gently.
v) Adding the resulting emulsion to a base formulation.

The emulsifier may be any suitable surfactant, but is preferably a blend of cationic surfactant and fatty alcohol, present such that the weight ratio of emulsifier to beeswax is from 1:100 to 1:10, preferably 1:50 to 1:20. A preferred emulsifier system is cetyl trimethylammonium chloride with cetearyl alcohol at a weight ratio of from 1:5 to 5:1.

Preferably the weight ratio of beeswax to silicone polymer is from 4:1 to 1:2, more preferably from 2:1 to 1:1 in the composition.

### Silicone Polymer

The silicone polymer in compositions of the invention suitable has a viscosity at 25°C measured at a shear rate of at least 0.01Hz of 80,000 mm²/s , preferably of at least 1 million mm²/s, more preferably of at least 10 million mm²/s, even more preferably greater than 100 million mm²/s.

The silicone polymer may be based upon any suitable polydialkyl or polydiaryl siloxane, but is preferable based upon polydimethylsiloxane. The silicone polymer is preferably water-insoluble and non-volatile.

Suitably, the silicone polymer is present in compositions of the invention as discrete particles with a median diameter (D₅₀) of 50 micrometres or less, preferably 20 micrometres or less, more preferably 10 micrometres or less and even more preferably 1 micrometre or less.

In an alternative embodiment of the invention, the silicone polymer may be in the form of a microemulsion, with a particle median diameter of less than 0.15 micrometres.

Preferably the silicone polymer is used as a pre-formed emulsion which can be added to the rest of the composition. This avoids the need for high-shear mixing of the composition to form suitably-sized particles of the silicone polymer in the composition.

It is highly preferred if the silicone polymer is a copolymer of divinyldimethicone and dimethicone having the structure: 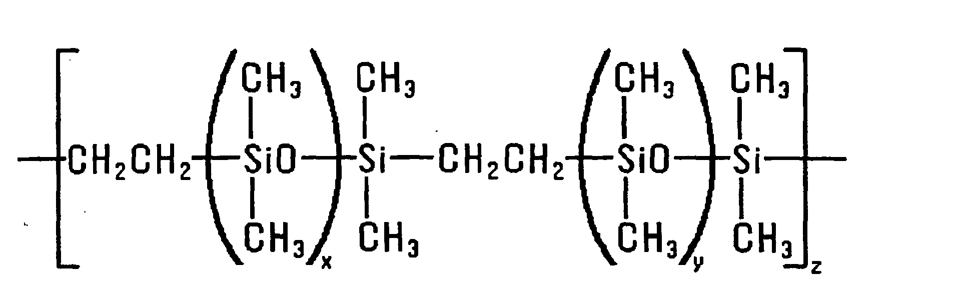 where x, y and z are all integers greater than 1. It is particularly preferred if the copolymer has a viscosity greater than 100 million mm²/s at a shear rate of 0.01 sec-1, and 25°C). A suitable commercial material supplied as an aqueous emulsion is Dow Corning HMW 2220.

The silicone polymer is suitably present as from 0.01% to 10% by weight of the composition, preferably from 0.1% to 5%, more preferably from 0.5% to 4%.

### Aqueous Hair Conditioning Compositions

Suitable compositions for the application of the invention include mousses, lotions and creams. Preferred are rinse-off hair conditioners. A particularly preferred composition is a cleansing shampoo or shower gel.

### Shampoos

In one particular aspect, compositions according to the invention are cleansing shampoos or shower gels which further comprise one or more cleansing surfactants which are cosmetically acceptable and suitable for topical application to the hair.

### Cleansing Surfactant

Suitable cleansing surfactants, which may be used singularly or in combination, are selected from anionic, nonionic, amphoteric and zwitterionic surfactants, and mixtures thereof. Mixtures of anionic and amphoteric surfactants are preferred.

### Anionic Cleansing Surfactant

Shampoo compositions according to the invention will typically comprise one or more anionic cleansing surfactants which are cosmetically acceptable and suitable for topical application to the hair.

Examples of suitable anionic cleansing surfactants are the alkyl sulphates, alkyl ether sulphates, alkaryl sulphonates, alkanoyl isethionates, alkyl succinates, alkyl sulphosuccinates, N-alkyl sarcosinates, alkyl phosphates, alkyl ether phosphates, alkyl ether carboxylates, and alphaolefin sulphonates, especially their sodium, magnesium, ammonium and mono-, di- and triethanolamine salts. The alkyl and acyl groups generally contain from 8 to 18 carbon atoms and may be unsaturated. The alkyl ether sulphates, alkyl ether phosphates and alkyl ether carboxylates may contain from 1 to 10 ethylene oxide or propylene oxide units per molecule.

Typical anionic cleansing surfactants for use in shampoo compositions of the invention include sodium oleyl succinate, ammonium lauryl sulphosuccinate, ammonium lauryl sulphate, sodium dodecylbenzene sulphonate, triethanolamine dodecylbenzene sulphonate, sodium cocoyl isethionate, sodium lauryl isethionate and sodium N-lauryl sarcosinate. The most preferred anionic surfactants are sodium lauryl sulphate, sodium lauryl ether sulphate(n)EO, (where n ranges from 1 to 3), ammonium lauryl sulphate and ammonium lauryl ether sulphate(n)EO, (where n ranges from 1 to 3).

Mixtures of any of the foregoing anionic cleansing surfactants may also be suitable.

The total amount of anionic cleansing surfactant in shampoo compositions of the invention is generally from 0.5 to 45, preferably from 1.5 to 35, more preferably from 5 to 20 percent by weight of the composition.

### Co-surfactant

The composition can include co-surfactants, to help impart aesthetic, physical or cleansing properties to the composition.

A preferred example is an amphoteric or zwitterionic surfactant, which can be included in an amount ranging from 0 to about 8, preferably from 1 to 4 percent by weight.

Examples of amphoteric and zwitterionic surfactants include alkyl amine oxides, alkyl betaines, alkyl amidopropyl betaines, alkyl sulphobetaines (sultaines), alkyl glycinates, alkyl carboxyglycinates, alkyl amphopropionates, alkylamphoglycinates, alkyl amidopropyl hydroxysultaines, acyl taurates and acyl glutamates, wherein the alkyl and acyl groups have from 8 to 19 carbon atoms. Typical amphoteric and zwitterionic surfactants for use in shampoos of the invention include lauryl amine oxide, cocodimethyl sulphopropyl betaine and preferably lauryl betaine, cocamidopropyl betaine and sodium cocamphopropionate.

Another preferred example is a nonionic surfactant, which can be included in an amount ranging from 0 to 8, preferably from 2 to 5 percent by weight of the composition.

For example, representative nonionic surfactants that can be included in shampoo compositions of the invention include condensation products of aliphatic (C8-C18) primary or secondary linear or branched chain alcohols or phenols with alkylene oxides, usually ethylene oxide and generally having from 6 to 30 ethylene oxide groups.

Other representative nonionic surfactants include mono- or di-alkyl alkanolamides. Examples include coco mono- or diethanolamide and coco mono-isopropanolamide.

Further nonionic surfactants which can be included in shampoo compositions of the invention are the alkyl polyglycosides (APGs). Typically, the APG is one which comprises an alkyl group connected (optionally via a bridging group) to a block of one or more glycosyl groups. Preferred APGs are defined by the following formula:
RO - (G)n
wherein R is a branched or straight chain alkyl group which may be saturated or unsaturated and G is a saccharide group. R may represent a mean alkyl chain length of from about C5 to about C20. Preferably R represents a mean alkyl chain length of from about C8 to about C12. Most preferably the value of R lies between about 9.5 and about 10.5. G may be selected from C5 or C6 monosaccharide residues, and is preferably a glucoside. G may be selected from the group comprising glucose, xylose, lactose, fructose, mannose and derivatives thereof. Preferably G is glucose.

The degree of polymerisation, n, may have a value of from about 1 to about 10 or more. Preferably, the value of n lies in the range of from about 1.1 to about 2. Most preferably the value of n lies in the range of from about 1.3 to about 1.5.

Suitable alkyl polyglycosides for use in the invention are commercially available and include for example those materials identified as: Oramix NS10 ex Seppic; Plantaren 1200 and Plantaren 2000 ex Henkel.

Other sugar-derived nonionic surfactants which can be included in compositions of the invention include the C10-C18 N-alkyl (C1-C6) polyhydroxy fatty acid amides, such as the C12-C18 N-methyl glucamides, as described for example in WO 92 06154 and US 5 194 639, and the N-alkoxy polyhydroxy fatty acid amides, such as C10-C18 N-(3-methoxypropyl) glucamide.

The composition according to the invention can also optionally include one or more cationic co-surfactants included in an amount from 0.01 to 10, more preferably from 0.05 to 5, most preferably from 0.05 to 2 percent by weight of the composition.

The total amount of cleansing surfactant (including any co-surfactant, and/or any emulsifier) in compositions of the invention is generally from 1 to 25, preferably from 2 to 20, more preferably from 5 to 17 percent by weight of the composition.

A preferred blend of cleansing surfactants is a combination of ammonium lauryl ether sulphate, ammonium lauryl sulphate, PEG 5 cocamide and cocamide MEA (CTFA designations).

### Cationic deposition polymers

A cationic polymer is a preferred ingredient in shampoo compositions of the invention, for enhancing conditioning performance of the shampoo, if the median particle size of the beeswax or of the silicone polymer is 10 micrometres or less.

The cationic polymer may be a homopolymer or be formed from two or more types of monomers. The molecular weight of the polymer will generally be between 5 000 and 10 000 000 Dalton, typically at least 10 000 and preferably from 100 000 to 2 000 000. The polymers will have cationic nitrogen containing groups such as quaternary ammonium or protonated amino groups, or a mixture thereof.

The cationic nitrogen-containing group will generally be present as a substituent on a fraction of the total monomer units of the cationic polymer. Thus when the polymer is not a homopolymer it can contain spacer non-cationic monomer units. Such polymers are described in the CTFA Cosmetic Ingredient Directory, 3rd edition. The ratio of the cationic to non-cationic monomer units is selected to give a polymer having a cationic charge density in the required range.

Suitable cationic conditioning polymers include, for example, copolymers of vinyl monomers having cationic amine or quaternary ammonium functionalities with water soluble spacer monomers such as (meth)acrylamide, alkyl and dialkyl (meth)acrylamides, alkyl (meth)acrylate, vinyl caprolactone and vinyl pyrrolidine. The alkyl and dialkyl substituted monomers preferably have C1-C7 alkyl groups, more preferably C1-3 alkyl groups. Other suitable spacers include vinyl esters, vinyl alcohol, maleic anhydride, propylene glycol and ethylene glycol.

The cationic amines can be primary, secondary or tertiary amines, depending upon the particular species and the pH of the composition. In general secondary and tertiary amines, especially tertiary, are preferred.

Amine substituted vinyl monomers and amines can be polymerized in the amine form and then converted to ammonium by quaternization.

The cationic conditioning polymers can comprise mixtures of monomer units derived from amine- and/or quaternary ammonium-substituted monomer and/or compatible spacer monomers.

Suitable cationic conditioning polymers include, for example:
- copolymers of 1-vinyl-2-pyrrolidine and 1-vinyl-3-methyl-imidazolium salt (e.g. chloride salt), referred to in the industry by the Cosmetic, Toiletry, and Fragrance Association, (CTFA) as Polyquaternium-16. This material is commercially available from BASF Wyandotte Corp. (Parsippany, NJ, USA) under the LUVIQUAT tradename (e.g. LUVIQUAT FC 370);
- copolymers of 1-vinyl-2-pyrrolidine and dimethylaminoethyl methacrylate, referred to in the industry (CTFA) as Polyquaternium-11. This material is available commercially from Gaf Corporation (Wayne, NJ, USA) under the GAFQUAT tradename (e.g., GAFQUAT 755N);
- cationic diallyl quaternary ammonium-containing polymers including, for example, dimethyldiallyammonium chloride homopolymer and copolymers of acrylamide and dimethyldiallylammonium chloride, referred to in the industry (CTFA) as Polyquaternium 6 and Polyquaternium 7, respectively;
- mineral acid salts of amino-alkyl esters of homo-and copolymers of unsaturated carboxylic acids having from 3 to 5 carbon atoms, (as described in U.S. Patent 4,009,256);
- cationic polyacrylamides(as described in WO95/22311).

Other cationic conditioning polymers that can be used include cationic polysaccharide polymers, such as cationic cellulose derivatives, cationic starch derivatives, and cationic guar gum derivatives. Suitably, such cationic polysaccharide polymers have a charge density from 0.1 to 4 meq/g.

Cationic polysaccharide polymers suitable for use in compositions of the invention include those of the formula:

A-O-[R-N⁺(R¹)(R²) (R³)X⁻],

wherein: A is an anhydroglucose residual group, such as a starch or cellulose anhydroglucose residual. R is an alkylene, oxyalkylene, polyoxyalkylene, or hydroxyalkylene group, or combination thereof. R¹, R² and R³ independently represent alkyl, aryl, alkylaryl, arylalkyl, alkoxyalkyl, or alkoxyaryl groups, each group containing up to about 18 carbon atoms. The total number of carbon atoms for each cationic moiety (i.e., the sum of carbon atoms in R¹, R² and R³) is preferably about 20 or less, and X is an anionic Counterion.

Cationic cellulose is available from Amerchol Corp. (Edison, NJ, USA) in their Polymer JR (trade mark) and LR (trade mark) series of polymers, as salts of hydroxyethyl cellulose reacted with trimethyl ammonium substituted epoxide, referred to in the industry (CTFA) as Polyquaternium 10. Another type of cationic cellulose includes the polymeric quaternary ammonium salts of hydroxyethyl cellulose reacted with lauryl dimethyl ammonium-substituted epoxide, referred to in the industry (CTFA) as Polyquaternium 24. These materials are available from Amerchol Corp. (Edison, NJ, USA) under the tradename Polymer LM-200.

Other suitable cationic polysaccharide polymers include quaternary nitrogen-containing cellulose ethers (e.g. as described in U.S. Patent 3,962,418), and copolymers of etherified cellulose and starch (e.g. as described in U.S. Patent 3,958,581).

A particularly suitable type of cationic polysaccharide polymer that can be used is a cationic guar gum derivative, such as guar hydroxypropyltrimonium chloride (commercially available from Rhone-Poulenc in their JAGUAR trademark series).

Examples are JAGUAR C13S, which has a low degree of substitution of the cationic groups and high viscosity. JAGUAR C15, having a moderate degree of substitution and a low viscosity, JAGUAR C17 (high degree of substitution, high viscosity), JAGUAR C16, which is a hydroxypropylated cationic guar derivative containing a low level of substituent groups as well as cationic quaternary ammonium groups, and JAGUAR 162 which is a high transparency, medium viscosity guar having a low degree of substitution.

Preferably the cationic conditioning polymer is selected from cationic cellulose and cationic guar derivatives. Particularly preferred cationic polymers are JAGUAR C13S, JAGUAR C15, JAGUAR C17 and JAGUAR C16 and JAGUAR C162.

The cationic conditioning polymer will generally be present in compositions of the invention at levels of from 0.01 to 5, preferably from 0.02 to 1, more preferably from 0.04 to 0.5 percent by weight of the composition.

### Rinse-off conditioner compositions

An alternative embodiment of the invention is as a rinse-off or leave-on conditioning composition.

Rinse-off or leave-on hair conditioner compositions according to the invention preferably comprise one or more conditioning surfactants which are cosmetically acceptable and suitable for topical application to the hair.

### Conditioning Surfactant

Suitable conditioning surfactants are selected from cationic surfactants, used singly or in admixture. Cationic surfactants useful in compositions of the invention contain amino or quaternary ammonium hydrophilic moieties which are positively charged when dissolved in the aqueous composition of the present invention.

Examples of suitable cationic surfactants are those corresponding to the general formula:

[N(R₁)(R₂)(R₃)(R₄)]⁺(X)⁻

in which R₁, R₂, R₃, and R₄ are independently selected from (a) an aliphatic group of from 1 to 22 carbon atoms, or (b) an aromatic, alkoxy, polyoxyalkylene, alkylamido, hydroxyalkyl, alkoylalkyl, aryl or alkylaryl group having up to 22 carbon atoms; and X is a salt-forming anion such as those selected from halogen, (e.g. chloride, bromide), acetate, citrate, lactate, glycolate, phosphate nitrate, sulphate, and alkylsulphate radicals.

The aliphatic groups can contain, in addition to carbon and hydrogen atoms, ether linkages, and other groups such as amino groups. The longer chain aliphatic groups, e.g., those of about 12 carbons, or higher, can be saturated or unsaturated.

Preferred cationic surfactants for conditioner compositions of the present invention are so-called monoalkyl quaternary ammonium compounds in which R₁ has an alkyl chain length from C16 to C22 and R₂, R₃ and R₄ have 2 or less carbon atoms.

Other preferred cationic surfactants are so-called dialkyl quaternary ammonium compounds in which R₁ and R₂ independently have an alkyl chain lengths from C16 to C22 and R₃ and R₄ have 2 or less carbon atoms.

Examples of suitable cationic surfactants include quaternary ammonium compounds, particularly trimethyl quaternary compounds.

Preferred quaternary ammonium compounds include cetyltrimethylammonium chloride, behenyltrimethylammonium chloride (BTAC), cetylpyridinium chloride, tetramethylammonium chloride, tetraethylammonium chloride, octyltrimethylammonium chloride, dodecyltrimethylammonium chloride, hexadecyltrimethylammonium chloride, octyldimethylbenzylammonium chloride, decyldimethylbenzylammonium chloride, stearyldimethylbenzylammonium chloride, didodecyldimethylammonium chloride, dioctadecyldimethylammonium chloride, tallowtrimethylammonium chloride, cocotrimethylammonium chloride, PEG-2 oleylammonium chloride and salts of these, where the chloride is replaced by halogen, (e.g., bromide), acetate, citrate, lactate, glycolate, phosphate nitrate, sulphate, or alkylsulphate. Further suitable cationic surfactants include those materials having the CTFA designations Quaternium-5, Quaternium-31 and Quaternium-18. Mixtures of any of the foregoing materials may also be suitable. Particularly useful quaternary ammonium cationic surfactants for use in hair conditioners of the invention are cetyltrimethylammonium chloride, available commercially, for example as GENAMIN CTAC, ex Hoechst Celanese and Arquad 16/29 supplied by Akzo Nobel, and behenyltrimethylammonium chloride (BTAC) such as Genamin KDM-P supplied by Clariant.

Another suitable cationic conditioning surfactant is a dialkoylalkyl dimethylammonium halide. An example of such a compound has the CTFA designation dipalmitoyethyldimethylammonium chloride.

Further suitable cationic systems are primary, secondary, and tertiary fatty amines used in combination with an acid to provide the cationic species. The alkyl groups of such amines preferably have from 12 to 22 carbon atoms, and can be substituted or unsubstituted. Particularly useful are amido substituted tertiary fatty amines, in particular tertiary amines having one C₁₂ to C₂₂ alkyl or alkenyl chain. Such amines, useful herein, include stearamidopropyIdimethylamine, stearamidopropyidiethylamine, stearamidoethyldiethylamine, stearamidoethyldimethylamine, palmitamidopropyld imethylamine, palmitamidopropyldiethylamine, palmitamidoethyldiethylamine, palmitamidoethyldimethylamine, behenamidopropyldimethylamine, behenamidopropyldiethylamine, behenamidoethyldiethylamine, behenamidoethyldimethylamine, arachidamidopropyldimethylamine, arachidamidopropyldiethylamine, arachidamidoethyldiethylamine, arachidamidoethyldimethylamine, diethylaminoethylstearamide.

Also useful are dimethylstearamine, dimethylsoyamine, soyamine, myristylamine, tridecylamine, ethylstearylamine, N-tallowpropane diamine, ethoxylated (with 5 moles of ethylene oxide) stearylamine, dihydroxyethylstearylamine, and arachidyl behenylamine.

As stated previously, these amines are typically used in combination with an acid to provide the cationic species. The preferred acid useful herein includes L- glutamic acid, lactic acid, hydrochloric acid, malic acid, succinic acid, acetic acid, fumaric acid, tartaric acid, citric acid, L-glutamic hydrochloride, and mixtures thereof; more preferably L-glutamic acid, lactic acid, citric acid. Cationic amine surfactants included among those useful in the present invention are disclosed in U.S. Patent 4,275,055 to Nachtigal, et al., issued June 23, 1981.

The molar ratio of protonatable amines to H⁺ from the acid is preferably from about 1:0.3 to 1:1.2, and more preferably from about 1:0.5 to about 1:1.1.

In the conditioners of the invention, the level of cationic surfactant is preferably from 0.01 to 10, more preferably 0.05 to 5, most preferably 0.1 to 4 percent by weight of the total composition.

### Fatty Materials

Conditioner compositions of the invention preferably additionally comprise fatty materials. The combined use of fatty materials and cationic surfactants in conditioning compositions is believed to be especially advantageous, because this leads to the formation of a structured lamellar or liquid crystal phase, in which the cationic surfactant is dispersed.

By "fatty material" is meant a fatty alcohol, an alkoxylated fatty alcohol, a fatty acid or a mixture thereof.
Preferably, the alkyl chain of the fatty material is fully saturated.

Representative fatty materials comprise from 8 to 22 carbon atoms, more preferably 16 to 22. Examples of suitable fatty alcohols include cetyl alcohol, stearyl alcohol and mixtures thereof. The use of these materials is also advantageous in that they contribute to the overall conditioning properties of compositions of the invention.

Alkoxylated, (e.g. ethoxylated or propoxylated) fatty alcohols having from about 12 to about 18 carbon atoms in the alkyl chain can be used in place of, or in addition to, the fatty alcohols themselves. Suitable examples include ethylene glycol cetyl ether, polyoxyethylene (2) stearyl ether, polyoxyethylene (4) cetyl ether, and mixtures thereof.

The level of fatty material in conditioners of the invention is suitably from 0.01 to 15, preferably from 0.1 to 10, and more preferably from 0.5 to 4 percent by weight of the total composition. The weight ratio of cationic surfactant to fatty alcohol is suitably from 10:1 to 1:10, preferably from 4:1 to 1:8, optimally from 1:1 to 1:7, for example 1:3.

### Suspending Agents

Optionally, the compositions according to the invention may further comprise from 0.1 to 10 percent by weight, preferably from 0.6% to 6%, of a suspending agent. Suitable suspending agents are selected from polyacrylic acids, cross-linked polymers of acrylic acid, copolymers of acrylic acid with a hydrophobic monomer, copolymers of carboxylic acid-containing monomers and acrylic esters, cross-linked copolymers of acrylic acid and acrylate esters, heteropolysaccharide gums and crystalline long chain acyl derivatives. The long chain acyl derivative is desirably selected from ethylene glycol stearate, alkanolamides of fatty acids having from 16 to 22 carbon atoms and mixtures thereof. Ethylene glycol distearate and polyethylene glycol 3 distearate are preferred long chain acyl derivatives. Polyacrylic acid is available commercially as Carbopol 420, Carbopol 488 or Carbopol 493. Polymers of acrylic acid cross-linked with a polyfunctional agent may also be used, they are available commercially as Carbopol 910, Carbopol 934, Carbopol 940, Carbopol 941 and Carbopol 980. An example of a suitable copolymer of a carboxylic acid containing a monomer and acrylic acid esters is Carbopol 1342. All Carbopol (trade mark) materials are available from Goodrich.

Suitable cross-linked polymers of acrylic acid and acrylate esters are Pemulen TR1 or Pemulen TR2. A suitable heteropolysaccharide gum is xanthan gum, for example that available as Kelzan mu.

However, if the median diameter of the particles of the beeswax and of the silicone polymer is less than 10 micrometres, it is preferred if the composition is free of suspending agent, by which is meant that compositions of the invention comprise less than 0.01% by weight of suspending agent. This is because suspending agents may deposit onto the hair, leading to a coated feel for some users.

### Other ingredients

Compositions according to the invention may additionally contain other ingredients suitable for use in hair cleansing and conditioning compositions. Other hydrophobic, water-insoluble conditioning oils may be included in addition to those of the invention.

The compositions of the present invention may also contain adjuvants suitable for hair care. Generally such ingredients are included individually at a level of up to 2 percent by weight of the total composition.

Among suitable hair care adjuvants, are natural hair root nutrients, such as amino acids and sugars. Examples of suitable amino acids include arginine, cysteine, glutamine, glutamic acid, isoleucine, leucine, methionine, serine and valine, and/or precursors and derivatives thereof. The amino acids may be added singly, in mixtures, or in the form of peptides, e.g. di- and tripeptides. The amino acids may also be added in the form of a protein hydrolysate, such as a keratin or collagen hydrolysate. Suitable sugars are glucose, dextrose and fructose. These may be added singly or in the form of, e.g. fruit extracts. A particularly preferred combination of natural hair root nutrients for inclusion in compositions of the invention is isoleucine and glucose. A particularly preferred amino acid nutrient is arginine. Another suitable adjuvant is glycolic acid.

### Mode of Use

The compositions of the invention are primarily intended for topical application to the hair and/or scalp of a human subject in rinse-off or leave-on compositions. The compositions are used to provide straightening, reduced volume and/or fluffiness of hair style after the hair is dried. In order to achieve the benefits it is not necessary to dry the hair using a heated air hair-drying apparatus, and it is preferred if the hair is allowed to dry naturally after towelling and brushing.

The invention will be illustrated by the following nonlimiting Examples. Examples of the invention are illustrated by a number, comparative Examples are illustrated by a letter.

### Examples

### Mean Radial spacing measurement:

The mean radial spacing measurement is linked to the volume of the hair, which is related to the manageability of the hair. Thus a low mean radial spacing measurement indicates that the hair is easy to mange.

### Shampoo treatment:

2g/10" Hair switches were tethered and rinsed under a tap. Using 4-5 switches per treatment. 0.2 ml of shampoo was placed along the length of the switch and agitated for 30 seconds, followed by a rinse for 30 seconds. Again, 0.2 ml of shampoo was placed along the length of the switch and agitated for 30 seconds, followed by a rinse for 1 minute. The switches were combed through whilst suspended vertically from a clamp stand, then rinsed with a water bottle to pull all the fibres together and allowed to dry naturally overnight.

### Laser measurements:

Each switch was suspended vertically from a clamp stand and a 2 mW, λ= 632.8nm Helium-Neon laser shone perpendicular to the untouched switch, approximately 2" from the bottom of the switch, and the illuminated image recorded onto an optical disc using a 35mm camera.

The discrimination level was set for each image using a macro, (i.e. the threshold value for a clear image resulting in the no. of dots stored onto the disc). The "lcalc" macro was used to calculate the x,y co-ordinates of every dot on the image and to convert this file into a readable text file. Another macro in Excel applied a mathematical transformation on all the co-ordinates, to convert the co-ordinates from their apparent position relative to the camera to their actual position in the switch. These actual co-ordinates were used to calculate the mean radial distribution of all the co-ordinates away from the calculated centre, thus providing an indicator for the volume of the switch. the lower the number, the lower the volume.

The results are dependant on the measuring conditions and hair types. Each set of comparative experiments were therefore performed at the same time with the same hair type.

### Table 1

Weight % in table 1 refers to the actual active chemical in the composition, and not the dilute raw material.

| **Chemical name** | **Trade Name** | **Supplier** | **weight %** | | | |
|---|---|---|---|---|---|---|
| | | | A | B | C | 1 |
| Sodium laureth (2 EO) sulphate | Empicol ESB70 | Albright & Wilson | 14 | 14 | 14 | 14 |
| Coco amidopropyl betaine | Tegobetaine CK | Goldschmidt | 2 | 2 | 2 | 2 |
| Guar Hydroxypropyl Trimonium Chloride | Jaguar C13S | Rhone Poulenc | 0.2 | 0.2 | 0.2 | 0.2 |
| Polydimethylsiloxane¹ | DC1785 | Dow Corning | 2.0 | - | 3.5 | - |
| Divinyldimethicone/ dimethicone copolymer¹ | HMW2220 | Dow Corning | - | - | - | 2.0 |
| Beeswax phEUR | | Koster Keunen | - | 1.5 | - | 1.5 |
| Ethylene Glycol Distearate | PK3000AM | COGNIS | 1 | 1 | 1 | 1 |
| Formaldehyde | Formalin | Mallinkropt | 0.1 | 0.1 | 0.1 | 0.1 |
| Water | - | - | To 100 | To 100 | To 100 | To 100 |

Salon tests were carried out where 18 panellists had their hair washed by a trained hairdresser in the salon as follows: The hair was wetted and parted down the centre. 3g of shampoo was applied to each side of the head and the hair washed in the usual way using -12°French Hard water at a temperature of ∼40°C. The hair was rinsed and the process repeated. The hair was then towelled and blow-dried by the panellist. During washing, and on completion, the hairdresser assessed various attributes of the hair.

Example A was compared with Example B in one test and Example 1 in a second test.

Testing of comparative example B was not completed because of objections to the coated, greasy feel resulting from the product. For product 1 compared to product A, product 1 was preferred at the 90% significance level for the attributes of alignment, less fluffy, ease of handling, softness and smoothness. Both products A and 1 were considered equivalent for greasy feel.

Hence the product according to the invention is shown to give improved manageability.

The mean radial spacing mm Example 1 and C was measured as detailed below:

| | Example 1 | Example C |
|---|---|---|
| Mean radial | 13.79 | 11.22 |
| Spacing mm | | |

Example 1 had a significantly lower mean radial spacing than Example C and thus decreased the volume of the hair.

Examples C and 1 when tested on a sensory panel gave the following results:

| | Smoothness | Ease of Comb | Clean feel |
|---|---|---|---|
| Example C | 51.4 | 33.3 | 44.4 |
| Example 1 | 48.6 | 66.7 | 55.6 |

The above table demonstrates that for the Example of the invention good conditioning is achieved without any detrimental effects to the clean feel of the hair.

| **Chemical name** | **Trade Name** | **Supplier** | **Weight%** | | | | |
|---|---|---|---|---|---|---|---|
| | | | 1 | C | 3 | 5 | 6 |
| Sodium laureth (2 EO) sulphate | Empicol ESB70 | Albright & Wilson | 14 | 14 | 14 | 14 | 14 |
| Coco amidopropyl betaine | Tegobetaine CK | Goldschmidt | 2 | 2 | 2 | 2 | 2 |
| Guar Hydroxypropyl Trimonium Chloride | Jaguar C13S | Rhone Poulenc | 0.2 | 0.2 | 0.2 | 0.2 | 0.2 |
| Polydimethylsiloxane¹ | DC1668 | Dow Corning | | 2.0 | | | |
| Polydimethylsiloxane² | DC1785 | Dow Corning | | | 2.0 | | |
| Divinyldimethicone/ dimethicone copolymer³ | HMW2220 | Dow Corning | 2.0 | | | 2.0 | 2.0 |
| Beeswax phEUR | | Koster Keunen | 1.5 | 1.5 | 1.5 | 0.5 | 3.0 |
| Ethylene Glycol Distearate | PK3000AM | COGNIS | 1.0 | 1.0 | 1.0 | 1.0 | 1.0 |
| Formaldehyde | Formalin | Mallinkropt | 0.1 | 0.1 | 0.1 | 0.1 | |
| Water | - | | To 100 | to 100 | to 100 | to 100 | |

DC1668 has a viscosity of 60,000 mm²/s at 25°C.
DC1785 has a viscosity of 1 million mm²/s at 25°C.
HMW2220 has a viscosity of 120 million mm²/s at 25°C.

The mean radial spacing of Examples 1, 5 and 6 were as follows:

| Example | 1 | 5 | 6 | C | 3 |
|---|---|---|---|---|---|
| Mean radial spacing mm | 15.2 | 16.3 | 17.3 | 17.0 | 16.3 |

Thus the formulation with 1.5 wt% beeswax out performed the formulation with 0.5 wt% and 3 wt% beeswax.

The formulations with high viscosity silicones outperformed Example C having a lower viscosity silicone.

## Claims

1. An aqueous hair care composition comprising:
a) 0.1% to 8% by weight of the total composition of beeswax
b) 0.01% to 10 % by weight of the total composition of a silicone polymer with a viscosity at 0.01Hz of at least 80,000 mm²/s at 25°C.

2. An aqueous hair care composition according to claim 1 in which the viscosity of the silicone at 0.01Hz is at least 1 million mm²/s at 25°C.

3. An aqueous hair care composition according to claim 1 or claim 2 in which the viscosity of the silicone at 0.01Hz is at least 100 million mm²/s at 25°C.

4. An aqueous hair care composition according to any preceding claim in which the level of silicone is from 0.5% to 4% by weight of the total composition.

5. An aqueous hair care composition according to any preceding claim in which the level of beeswax is from 0.2% to 5% by weight of the total composition.

6. A composition according to any preceding claim wherein the beeswax and the silicone polymer are present in the form of particles with a median diameter (D₅₀) of 50 micrometres or less.

7. A composition according to any preceding claim wherein the composition is a cleansing composition further comprising from 1% to 25% by weight of a cleansing surfactant.

8. A composition according to any preceding claim wherein the cleansing surfactant is selected from the group consisting of anionic surfactants, amphoteric surfactants, zwitterionic surfactants, nonionic surfactants and mixtures thereof.

9. A process for preparing the hair care composition described in any one of the above claims comprising the step of emulsifying the beeswax before addition to the composition.

10. A process according to claim 9 in which the beeswax is emulsified with a cationic surfactant.

11. Use of the composition of any of the preceding claims for conditioning hair.

## Patentansprüche

1. Wässrige Haarpflegezusammensetzung, umfassend:
a) 0,1 Gewichts-% bis 8 Gewichts-%, bezogen auf die gesamte Zusammensetzung, Bienenwachs,
b) 0,01 Gewichts-% bis 10 Gewichts-%, bezogen auf die gesamte Zusammensetzung, eines Silicon-Polymers mit einer Viskosität bei 0,01 Hz von wenigstens 80 000 mm²/s bei 25 °C.

2. Wässrige Haarpflegezusammensetzung gemäß Anspruch 1, in der die Viskosität des Silicons bei 0,01 Hz wenigstens 1 Million mm²/s bei 25 °C ist.

3. Wässrige Haarpflegezusammensetzung gemäß Anspruch 1 oder Anspruch 2, in der die Viskosität des Silicons bei 0,01 Hz wenigstens 100 Millionen mm²/s bei 25 °C ist.

4. Wässrige Haarpflegezusammensetrung gemäß einem vorangehenden Anspruch, in der die Siliconkonzentration von 0,5 Gewichts-% bis 4 Gewichts-%, bezogen auf die gesamte Zusammensetzung, ist.

5. Wässrige Haarpflegezusammensetzung gemäß einem vorangehenden Anspruch, in der die Bienenwachskonzentration von 0,2 Gewichts-% bis 5 Gewichts-%, bezogen auf die gesamte Zusammensetzung, ist.

6. Zusammensetzung gemäß einem vorangehenden Anspruch, wobei das Bienenwachs und das Silicon-Polymer in der Form von Partikeln mit einem mittleren Durchmesser (D₅₀) von 50 Mikrometer oder weniger vorliegen.

7. Zusammensetzung gemäß einem vorangehenden Anspruch, wobei die Zusammensetzung eine Reinigungszusammensetzung ist, die außerdem von 1 Gewichts-% bis 25 Gewichts-% eines Reinigungstensids umfasst.

8. Zusammensetzung gemäß einem vorangehenden Anspruch, wobei das Reinigungstensid ausgewählt ist aus der Gruppe, bestehend aus anionischen Tensiden, amphoteren Tensiden, zwitterionischen Tensiden, nicht-ionischen Tensiden und Gemischen davon.

9. Verfahren zur Herstellung der Haarpflegezusammensetzung, die in einem der obigen Ansprüche beschrieben ist, umfassend den Schritt eines Emulgierens des Bienenwachses vor Zugabe zu der Zusammensetzung.

10. Verfahren gemäß Anspruch 9, wobei das Bienenwachs mit einem kationischen Tensid emulgiert wird.

11. Verwendung der Zusammensetzung gemäß einem der vorangehenden Ansprüche zur Konditionierung von Haar.

## Revendications

1. Composition aqueuse de soin capillaire comprenant :
a) de 0,1 % à 8 % en poids de la composition totale de cire d'abeille
b) de 0,01 % à 10 % en poids de la composition totale d'un polymère de silicone ayant une viscosité à 0,01 Hz d'au moins 80 000 mm²/s à 25°C.

2. Composition aqueuse de soin capillaire selon la revendication 1 dans laquelle la viscosité de la silicone à 0,01 Hz est d'au moins 1 million mm²/s à 25°C.

3. Composition aqueuse de soin capillaire selon la revendication 1 ou la revendication 2 dans laquelle la viscosité de la silicone à 0,01 Hz est d'au moins 100 millions mm²/s à 25°C.

4. Composition aqueuse de soin capillaire selon l'une des revendications précédentes dans laquelle la teneur en silicone se situe dans la plage allant de 0,5 % à 4 % en poids de la composition totale.

5. Composition aqueuse de soin capillaire selon l'une des revendications précédentes dans laquelle la teneur en cire d'abeille se situe dans la plage allant de 0,2 % à 5 % en poids de la composition totale.

6. Composition selon l'une des revendications précédentes dans laquelle la cire d'abeille et le polymère de silicone sont présents sous forme de particules ayant un diamètre médian (D₅₀) de 50 micromètres ou moins.

7. Composition selon l'une des revendications précédentes dans laquelle la composition est une composition nettoyante comprenant en outre de 1 % à 25 % en poids d'un tensioactif nettoyant.

8. Composition selon l'une des revendications précédentes dans laquelle le tensioactif nettoyant est choisi parmi le groupe constitué par les tensioactifs anioniques, les tensioactifs amphotériques, les tensioactifs zwitterioniques, les tensioactifs non-ioniques et les mélanges de ceux-ci.

9. Procédé de préparation de la composition de soin capillaire décrite dans l'une quelconque des revendications précédentes comprenant l'étape consistant à émulsionner la cire d'abeille avant de l'ajouter à la composition.

10. Procédé selon la revendication 9 dans laquelle la cire d'abeille est émulsionnée avec un tensioactif cationique.

11. Utilisation de la composition selon l'une des revendications précédentes comme soin capillaire.
